# EUROPEAN PATENT APPLICATION

(11) **EP 3 461 480 A1**
(43) Date of publication of application: **03.04.2019**
(21) Application number: 17193567.9
(22) Date of filing: 27.09.2017
(51) Int. Cl.: A61K 31/18, A61K 31/4965, A61K 31/497, A61P 35/00

(54) **COMBINATION OF A DNA DAMAGE RESPONSE CELL CYCLE CHECKPOINT INHIBITORS AND BELINOSTAT FOR TREATING CANCER**

(71) Applicant: ONXEO, 75015 Paris (FR)
(72) Inventor: The designation of the inventor has not yet been filed
(74) Representative: Simon, Alexandre

(57) **Abstract**

The present invention relates to the combination of a DNA damage response (DDR) cell cycle checkpoint inhibitor selected from the group consisting of an ATR inhibitor (ATRi) and a CHK1 inhibitor (CHKli) and belinostat for treating cancer.

## Description

### Field of the Invention

The present invention relates to the field of medicine, in particular of oncology.

### Background of the Invention

ATR ("ataxia telangiectasia and Rad-3-related") is a protein kinase involved in cellular responses to DNA damage, commonly referred to as the DNA Damage Response ("DDR"). ATR along with its substrates coordinates multiple cell functions including cell cycle control, DNA replication and DNA damage repair. CHK1 ("checkpoint kinase 1"), a protein kinase which is required for checkpoint-mediated cell cycle arrest and activation of DNA repair in response to the presence of DNA damage, is a principle substrate for ATR.

ATR inhibitors and CHK1 inhibitors have been shown to reduce cell survival in some cancer cells. Additionally, these inhibitors have been shown to sensitise some cancer cells to the cytotoxic effects of multiple DNA damaging drugs and ionizing radiation.

At the same time, belinostat has been shown to exert multiple cytotoxic actions in cancer cells. This observation led to the development of belinostat to treat cancers. However, despite its preclinical potential, the clinical use of belinostat remains restricted to a certain subset of T-cell lymphoma. Belinostat (PXD-101; Beleodaq® Spectrum Pharmaceuticals, Inc., USA) is only approved for peripheral T-cell lymphoma (PTCL). The use of belinostat is thus limited to particular tumor types and cannot be used for treating any cancer including solid tumors.

There remains a substantial unmet need for therapeutics that have better therapeutic efficacy, longer clinical benefit, and improved safety profiles, particularly for those patients with cancers that are resistant to existing therapeutics such as triple-negative breast cancer (TNBC).

### Summary of the Invention

The present invention provides a combined treatment allowing to use belinostat for treating any kind of cancers, in particular for solid cancer, especially resistant solid cancer such as triple-negative breast cancer (TNBC) without displaying toxicity towards normal cells.

The present invention provides a combination of belinostat with a DNA damage response (DDR) cell cycle checkpoint inhibitor selected from the group consisting of an ATR inhibitor (ATRi) and a CHK1 inhibitor (CHK1i), leading to a synergistic effect against tumors.

The present invention relates to a pharmaceutical composition or a kit (kit-of-parts) comprising belinostat with a DDR cell cycle checkpoint inhibitor selected from the group consisting of an ATRi and a CHK1i, in particular for use for treating cancer.

It further relates to a kit (kit-of-parts) comprising belinostat and a DDR cell cycle checkpoint inhibitor selected from the group consisting of an ATRi and a CHK1i as a combined preparation for simultaneous, separate or sequential use, in particular in the treatment of cancer.

Preferably, the ATR inhibitor is selected from the group consisting of VE-821, VE-822VX-970, AZD-6738, AZ20, and BAY-1895344.

Preferably, the CHK1 inhibitor is selected from the group consisting of LY2603618, LY2606368, SCH900776/MK-8776, SCH900444, AZD-7762, CHIR-124, PF-477736 SRA737 and GDC-0575.

In a particular aspect, the belinostat is used with a sub-therapeutic amount.

All cancer type can be treated. More preferably, the cancer is selected from leukemia, lymphoma, sarcoma, melanoma, and cancers of the head and neck, kidney, ovary, pancreas, prostate, thyroid, lung, in particular small-cell lung cancer, and non-small-cell lung cancer, esophagus, breast (TBNC), bladder, colorectum, liver, cervix, and endometrial and peritoneal cancers. In particular, the cancer is a solid cancer. In a particular aspect, the cancer is a metastatic cancer or high-grade or advanced cancer. In a particular embodiment, the cancer is selected from leukemia, lymphoma, melanoma, sarcoma, cancer of the head and neck, breast cancer (TBNC), brain cancer, colorectum cancer, and cancer of cervix.

### Brief Description of the Drawings

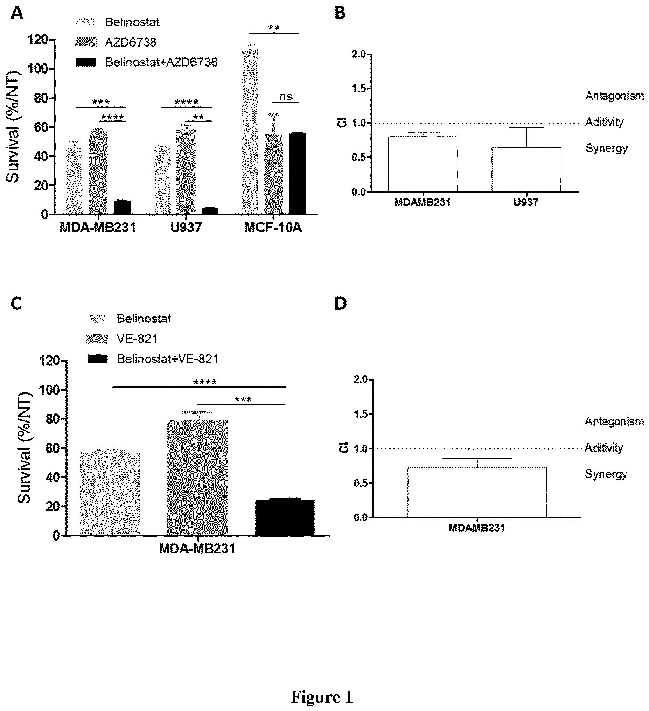

**Figure 1****. The combined treatment Belinostat and ATR inhibitors display synergistic efficacy. A, C.** Efficacy of belinostat, ATRi (AZD6738 **(A)** or VE-821 **(C))** and both (at doses corresponding to IC50) was monitored 7 days or 4 days after treatment by XTT assay. Survival is expressed as percentage of living cells relative to non-treated condition. Data are expressed as mean + SD of at least 4 independent cultures. **B, D.** Combination index (CI) analysis to determine synergy between belinostat and ATRi (AZD6738 **(B)** or VE-821 **(D))** was carried out using Calcusyn software as described in methods. CI<1 indicates synergy, CI=1 is additive, and CI>1 indicates antagonism. **, P<0.01; ***, P<0.001; ****, P<0.0001; ns, not significant.

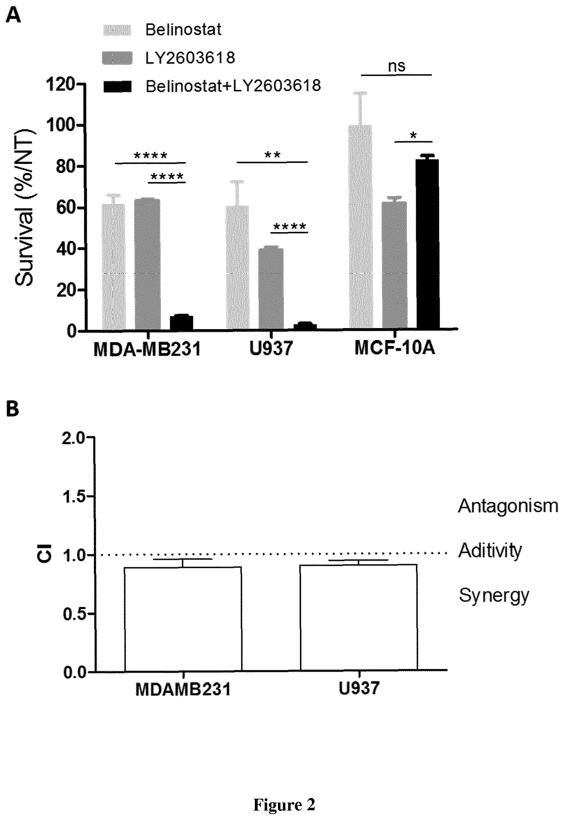

**Figure 2****. The combined treatment belinostat and CHK1 inhibitors display synergistic efficacy. A.** Efficacy of belinostat, CHK1i or both (at doses corresponding to IC50) was monitored 7 days or 4 days after treatment by XTT assay. Survival is expressed as percentage of living cells relative to non-treated condition. Data are expressed as mean + SD of at least 4 independent cultures. **B.** Combination index (CI) analysis to determine synergy between belinostat and CHK1i was carried out using Calcusyn software as described in methods. CI<1 indicates synergy, CI=1 is additive, and CI>1 indicates antagonism. *, P<0.05; **, P<0.01; ****, P<0.0001; ns, not significant.

### Detailed Description of the Invention

The present invention relates to a pharmaceutical composition or a kit (kit-of-parts) comprising belinostat and a DNA damage response (DDR) cell cycle checkpoint inhibitor selected from the group consisting of ATR inhibitor (ATRi) and CHK1 inhibitor (CHK1i).

The present invention relates to pharmaceutical composition comprising belinostat and a DDR cell cycle checkpoint inhibitor selected from the group consisting of an ATRi and a CHK1i for use in the treatment of a cancer.

The present invention also relates to kit (kit-of-parts) comprising belinostat and a DDR cell cycle checkpoint inhibitor selected from the group consisting of an ATRi and a CHK1i as a combined preparation for simultaneous, separate or sequential use, in particular for use in the treatment of cancer.

The present invention further relates to a method for treating a cancer in a subject in need thereof, comprising administering a therapeutically effective amount of belinostat and a therapeutically effective amount of a DDR cell cycle checkpoint inhibitor selected from the group consisting of an ATRi and a CHK1i, and optionally a pharmaceutically acceptable carrier.

The present invention further relates to a method for treating a cancer in a subject in need thereof, comprising administering a subtherapeutically effective amount of belinostat and a subtherapeutically effective amount of a DDR cell cycle checkpoint inhibitor selected from the group consisting of an ATRi and a CHK1i, and optionally a pharmaceutically acceptable carrier.

### Definitions:

The terms "kit", "product" or "combined preparation", as used herein, defines especially a "kit-of-parts" in the sense that the combination partners as defined above can be dosed independently or by use of different fixed combinations with distinguished amounts of the combination partners, i.e. simultaneously or at different time points. The parts of the kit-of-parts can then, e.g., be administered simultaneously or chronologically staggered, that is at different time points and with equal or different time intervals for any part of the kit of parts. The ratio of the total amounts of the combination partners to be administered in the combined preparation can be varied. The combination partners can be administered by the same route or by different routes.

Within the context of the invention, the term treatment denotes curative, symptomatic, preventive treatment as well as maintenance treatment. Pharmaceutical compositions, kits, products and combined preparations of the invention can be used in humans with existing cancer or tumor, including at early or late stages of progression of the cancer. The pharmaceutical compositions, kits, products and combined preparations of the invention will not necessarily cure the patient who has the cancer but will delay or slow the progression or prevent further progression of the disease, ameliorating thereby the patients' condition. In particular, the pharmaceutical compositions, kits, products and combined preparations of the invention reduce the development of tumors, reduce tumor burden, produce tumor regression in a mammalian host and/or prevent metastasis occurrence and cancer relapse. In treating the cancer, the pharmaceutical composition of the invention is administered in a therapeutically effective amount.

By "therapeutically effective amount" it is meant the quantity of the compound of interest of the pharmaceutical composition, kit, product or combined preparation of the invention which prevents, removes or reduces the deleterious effects of cancer in mammals, including humans, alone or in combination with the other active ingredients of the pharmaceutical composition, kit, product or combined preparation. It is understood that the administered dose may be lower for each compound in the composition to the "therapeutically effective amount" define for each compounds used alone or in combination with other treatments than the combination described here. The "therapeutically effective amount" of the composition will be adapted by those skilled in the art according to the patient, the pathology, the mode of administration, etc.

By "subtherapeutically effective amount" it is meant the quantity of the compound of interest of the pharmaceutical composition, kit, product or combined preparation of the invention which is effective at a lower dosage when used in the combination regimen of the invention, as compared to the dosage that is effective when used alone. Subtherapeutically effective amounts may be readily determined by one of skill in the art, in view of the teachings herein. Accordingly, as described herein, subtherapeutically effective amounts of belinostat and an ATRi inhibitor (such as AZD-6738) or a CHK1 inhibitor (such as LY2603618) may be used to achieve a therapeutic effect when administered in combination.

Whenever within this whole specification the terms "treatment of a cancer" or "treating a cancer" or the like are mentioned with reference to the pharmaceutical composition, kit, product or combined preparation of the invention, there is meant: a) a method for treating a cancer, said method comprising administering a pharmaceutical composition, kit, product or combined preparation of the invention to a subject in need of such treatment; b) the use of a pharmaceutical composition, kit, product or combined preparation of the invention for the treatment of a cancer; c) the use of a pharmaceutical composition, kit, product or combined preparation of the invention for the manufacture of a medicament for the treatment of a cancer; and/or d) a pharmaceutical composition, kit, product or combined preparation of the invention for use in the treatment a cancer.

The pharmaceutical compositions, kits, products or combined preparations contemplated herein may include a pharmaceutically acceptable carrier in addition to the active ingredient(s). The term "pharmaceutically acceptable carrier" is meant to encompass any carrier (e.g., support, substance, solvent, etc.) which does not interfere with effectiveness of the biological activity of the active ingredient(s) and that is not toxic to the host to which it is administered. For example, for parental administration, the active compounds(s) may be formulated in a unit dosage form for injection in vehicles such as saline, dextrose solution, serum albumin and Ringer's solution.

The pharmaceutical composition, kit, product or combined preparation can be formulated as solutions in pharmaceutically compatible solvents or as emulsions, suspensions or dispersions in suitable pharmaceutical solvents or vehicle, or as pills, tablets or capsules that contain solid vehicles in a way known in the art. Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. Formulations suitable for parental administration conveniently comprise a sterile oily or aqueous preparation of the active ingredient which is preferably isotonic with the blood of the recipient. Every such formulation can also contain other pharmaceutically compatible and nontoxic auxiliary agents, such as, e.g. stabilizers, antioxidants, binders, dyes, emulsifiers or flavouring substances. The formulations of the present invention comprise an active ingredient in association with a pharmaceutically acceptable carrier therefore and optionally other therapeutic ingredients. The carrier must be "acceptable" in the sense of being compatible with the other ingredients of the formulations and not deleterious to the recipient thereof. The pharmaceutical compositions, kits, products or combined preparations are advantageously applied by injection or intravenous infusion of suitable sterile solutions or as oral dosage by the digestive tract. Methods for the safe and effective administration of most of these chemotherapeutic agents are known to those skilled in the art. In addition, their administration is described in the standard literature.

### Belinostat:

Belinostat (also known as PXD-101) has the chemical name (2E)-N-hydroxy-3-[3-(phenylsulfamoyl)phenyl]prop-2-enamide and the following chemical formula:

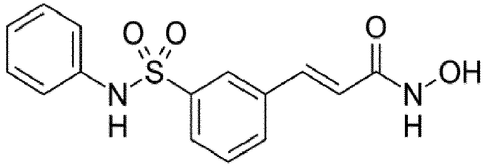

Belinostat is currently commercially available for injection in the U.S. under the brand name Beleodaq® (Spectrum Pharmaceuticals). Typically, liquid formulations of belinostat comprise L-arginine, and are suitable for administration by injection, infusion, intravenous infusion, etc

Belinostat and pharmaceuticals compositions comprising thereof useful in the present combinations are described in the international patent applications N° WO 2002/30879 and WO 2006/120456, the contents of both of which are incorporated herein in their entirety. In certain embodiments, belinostat is formulated with arginine (such as L-arginine).

Belinostat may be used under any pharmaceutically acceptable form, including without limitation, their free form and their pharmaceutically acceptable salts or solvates.

The term "pharmaceutically acceptable salts" refers to salts prepared from pharmaceutically acceptable, preferably non-toxic, bases or acids including mineral or organic acids or organic or inorganic bases. Such salts are also known as acid addition and base addition salts." Examples of pharmaceutically acceptable salts are discussed in Berge et al., 1977, "Pharmaceutically Acceptable Salts," J. Pharm. Sci., Vol. 66, pp. 1-19.

The term "solvate" refers to a molecular complex comprising the drug substance and a stoichiometric or non-stoichiometric amount of one or more pharmaceutically acceptable solvent molecules (e.g., ethanol). The term "hydrate" refers to a solvate comprising the drug substance and a stoichiometric or non-stoichiometric amount of water.

In another aspect the present invention relates to a pharmaceutical composition or a kit (kit-of-parts) comprising an HDAC inhibitor (HDACi) and an ATR inhibitor (ATRi). The present invention also relates to pharmaceutical composition comprising an HDACi and an ATRi for use in the treatment of a cancer. The present invention further relates to kit (kit-of-parts) comprising an HDACi and an ATRi as a combined preparation for simultaneous, separate or sequential use, in particular for use in the treatment of cancer.

Many HDAC inhibitors are known and, thus, can be synthesized by known methods from starting materials that are known, may be available commercially, or may be prepared by methods used to prepare corresponding compounds in the literature.

A preferred class of HDAC inhibitors are hydroxamic acid inhibitors which are disclosed e. g. in WO 97/35990, US-A 5, 369, 108, US-A 5, 608, 108, US-A 5, 700, 811, WO 01/18171, WO 98/55449, WO 93/12075, WO 01/49290, WO 02/26696, WO 02/26703, JP 10182583, WO 99/12884, WO 01/38322, WO 01/70675, WO 02/46144, WO 02/22577 and WO 02/30879. All HDAC inhibitors disclosed in these publications are included herein by reference.

Particularly preferred are HDAC inhibitors are carbamic acid compounds comprising a sulfonamide linkage which are disclosed e. g. in WO 02/30879.

Other HDAC inhibitors which can be included within the compositions of the present invention are cyclic peptide inhibitors, and here it can be referred e. g. to US-A 5,620, 953, US-A 5, 922, 837, WO 01/07042, WO 00/08048, WO 00/21979, WO 99/11659, WO 00/52033 and WO 02/0603. All HDAC inhibitors disclosed in these publications are included herein by reference.

Suitable HDAC inhibitors are also those which are based on a benzamide structure which are disclosed e. g. in Proc. Natl. Acad. Sci. USA (1999), 96: 4592-4597, but also in EP-A 847 992, US 6, 174, 905, JP 11269140, JP 11335375, JP 11269146, EP 974 576, WO 01/38322, WO 01/70675 and WO 01/34131. All HDAC inhibitors, which are disclosed in these documents, are included herein by reference.

In a preferred embodiment, the HDAC inhibitor is selected from the group consisting of belinostat (PXD-101), vorinostat (SAHA), entinostat (MS-275), romidepsin (FK-228), panobinostat (LBH-589), givinostat (IFT2357), abexinostat (PCI-24781), quisinostat (JNJ-26481585, pracinostat (SB939), tefinostat (CHR-2845) and dacinostat (LAQ-824).

### DDR:

The cellular DNA damage response (DDR) comprises signaling networks that regulate a spectrum of processes, including cell cycle progression, which enable DNA repair to occur. The DDR is believed to stimulate DNA repair, promote survival and stalls cell cycle progression by activating cell cycle checkpoints, which provide time for repair. Without the DDR, cells are much more sensitive to DNA damage and readily die from DNA lesions induced by endogenous cellular processes such as DNA replication or exogenous DNA damaging agents commonly used in cancer therapy.

### ATR inhibitors:

ATR ("ataxia telangiectasia and Rad-3-related") is a protein kinase involved in cellular responses to DNA damage. ATR is a protein kinase that maintains genome stability and halts cell cycle phase transitions in response to DNA lesions that block DNA polymerase movement. These DNA replication-associated features of ATR function have led to the emergence of ATR kinase inhibitors as potential adjuvants for DNA-damaging cancer chemotherapeutics.

An "ATR inhibitor" refers to any compound or substance that can inhibit the activity of ATR kinase. The ATR kinase activity can be determined by a variety of techniques well known by the skilled person. Usually, the basic mechanism of this assay is to observe the phosphorylated levels of a fragment of p53 containing the site that can be phosphorylated by ATR *in vitro* such described in PCT International Publication WO 2011/154737, the disclosure of which is hereby incorporated by reference in his entirety. These methods are ideal for the determination of IC50 values of known or suspected ATR inhibitors.

Many ATR inhibitors are known and, thus, can be synthesized by known methods from starting materials that are known, may be available commercially, or may be prepared by methods used to prepare corresponding compounds in the literature.

For instance, the compound is an ATR inhibitor selected from a compound described in PCT International Publications WO 2013/049726, WO 2013/152298, WO 2013/049859, US-2013-0089625, US-2013-0115312, US-2014-0107093, US-2013-0096139, WO 2011/143426, US-2013-0095193, WO 2014/055756, WO 2011/143419, WO 2011/143422, WO 2011/143425, US-2013-0115311, US-2013-0115312, US-2013-0115313, US-2013-0115314, WO 2011/163527, WO 2012/178123, WO 2012/178124, WO 2012/178125, US-2014-0113005, WO2013/049726, WO 2013/071085, WO 2010/071837, WO 2014/089379, WO 2014/143242, WO 2014/143241, WO 2015/084384 and/or WO 2014/143240.

In preferred embodiments, the ATR inhibitor is a compound of Formula A-1, A-2, A-3, or A-4 or a pharmaceutically acceptable salt thereof.
A-1 (VE-821) is also known as 3-amino-6-(4-methylsulfonylphenyl)-N-phenylpyrazine-2-carboxamide and has the following chemical structure: 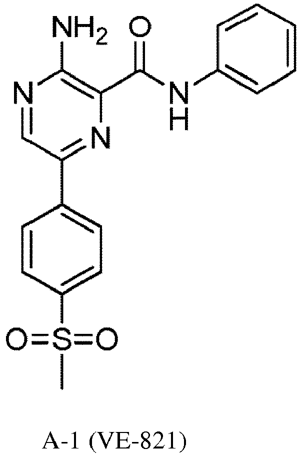
A-2 (VE-822/VX-970) is also known as 3-[3-[4-(methylaminomethyl)phenyl]-1,2-oxazol-5-yl]-5-(4-propan-2-ylsulfonylphenyl)pyrazin-2-amine and has the following chemical structure: 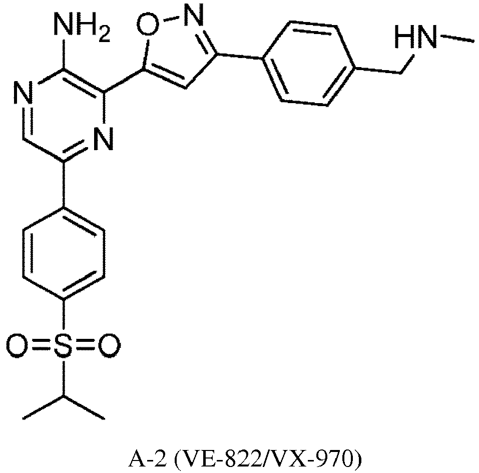
A-3 and has the following chemical structure: 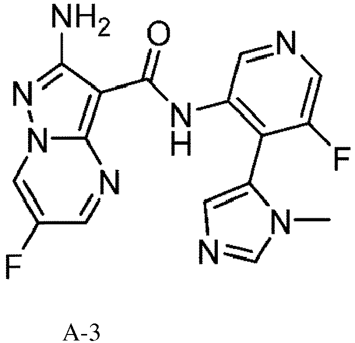
A-4 and has the following chemical structure:

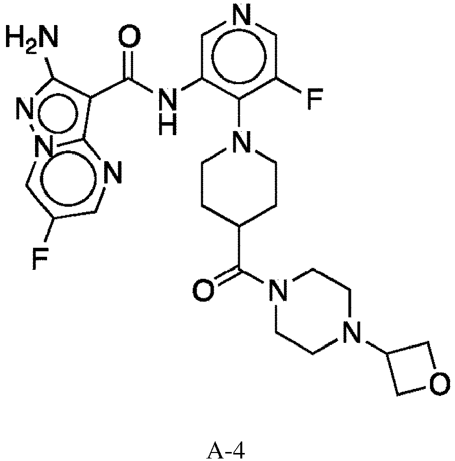

In another embodiment, the compound is an ATR inhibitor selected from a compound described in PCT International Publications WO 2015/187451, WO 2015/085132, WO 2014/062604, WO 2014/143240, WO 2013/071094, WO 2013/071093, WO 2013/071090, WO 2013/071088; WO 2013/049859, WO 2013/049719, WO 2013/049720, WO 2013/049722, WO 2012/138938, WO 2011/163527, WO 2011/143423, WO 2011/143399 and/or WO 2010/054398.

In yet another embodiment, the compound is an ATR inhibitor selected from a compound described in PCT International Publication WO 2011/154737.

In a preferred embodiment, the ATR inhibitor is AZD-6738 or a pharmaceutically acceptable salt thereof. AZD-6738 is also known as 4-[4-[1-[[S(R)]-S-methylsulfonimidoyl]cyclopropyl]-6-[(3R)-3-methyl-4-morpholinyl]-2-pyrimidinyl]-1H-pyrrolo[2,3-b]pyridine and has the following chemical structure:

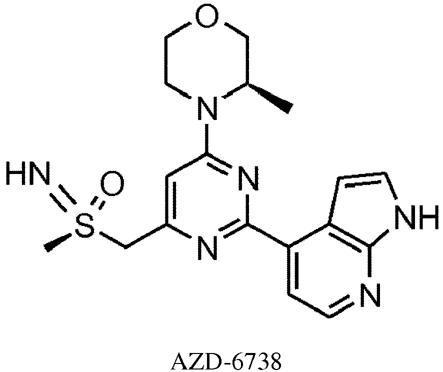

In a preferred embodiment, the ATR inhibitor is AZ20 or a pharmaceutically acceptable salt thereof. AZ20 is also known as 4-[4-[(3R)-3-Methyl-4-morpholinyl]-6-[1-(methylsulfonyl)cyclopropyl]-2-pyrimidinyl]-1H-indole and has the following chemical structure:

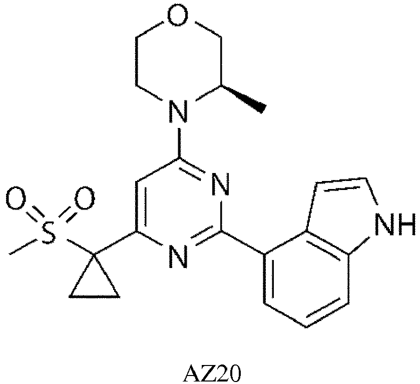

In yet another embodiment, the compound is an ATR inhibitor selected from a compound described in PCT International Publications WO 2013/142214 and WO 2017/011302.

In still yet another embodiment, the compound is an ATR inhibitor selected from a compound described in PCT International Publications WO 2016/020320 and WO 2017/121684.

In a preferred embodiment, the ATR inhibitor is BAY-1895344 or a pharmaceutically acceptable salt thereof. BAY-1895344 is also known as (R)-3-methyl-4-(4-(1-methyl-1H-pyrazol-5-yl)-8-(1H-pyrazol-5-yl)-1,7-naphthyridin-2-yl)morpholine and has the following chemical structure:

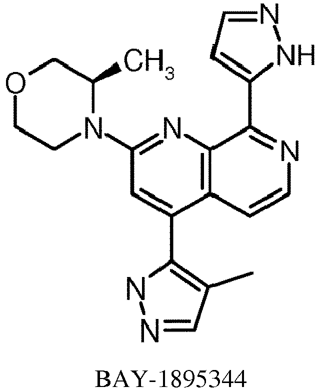

In a particular embodiment, the ATR inhibitor is selected from the group consisting of VE-821, VE-822 (also known as VX-970), AZD-6738and BAY-1895344.

### CHK1 inhibitors

CHK1 is a protein kinase that lies downstream from ATR in the DNA damage checkpoint signal transduction pathway. In mammalian cells, CHK1 is phosphorylated in response to agents that cause DNA damage including ionizing radiation (IR), ultraviolet (UV) light, and hydroxyurea. This phosphorylation which activates CHK1 in mammalian cells is dependent on ATR. CHK1 plays a role in the ATR dependent DNA damage checkpoint leading to arrest in S phase and at G2M and thus preventing cells from entering mitosis in the presence of DNA damage.
A "CHK1 inhibitor" refers to any compound or substance that can inhibit the activity of CHK1 kinase. The CHK1 kinase activity can be determined by a variety of techniques well known by the skilled person. Usually, the basic mechanism of this assay is to observe the phosphorylated levels of a fragment of CDC25C containing the site that can be phosphorylated by CHK1 *in vitro.* Commercial kits for such techniques are available (see for example, abcam's kits with using an enzyme-based immunoassay). Other methods may also be used such as the one developed by Zabludoff S. et al (Mol Cancer Ther 2008;7(9). September 2008), the disclosure of which is hereby incorporated by reference in his entirety. These methods are ideal for the determination of IC50 values of known or suspected CHK1 inhibitors.

Many CHK1 inhibitors are known and, thus, can be synthesized by known methods from starting materials that are known, may be available commercially, or may be prepared by methods used to prepare corresponding compounds in the literature.

See for example, PCT International Publications WO 02/070494, WO 03/028724, WO 03/029241, WO 03/028731, WO 03/093297, WO 04/063198, WO 04/081008; WO 05/009435, WO 05/016909, WO 05/066163 WO 2005/103036, WO 2006/120573, WO 2006/106326, WO 2007/090494, WO 2007/090493, WO 2008/012635, WO 2009/004329, and those described in Prudhomme, Michelle, Novel checkpoint 1 inhibitors, Recent Patents on Anti-Cancer Drug Discovery (2006), 1(1), 55-68 and Lainchbury M, Collins I. Checkpoint kinase inhibitors. A patent review (2009-2010) Exp Opin Ther Patents. 2011;21:1191-210.

In one embodiment, the compound is a CHK1 inhibitor selected from a compound described in PCT International Publications WO 2006/105262, WO 2010/077758 and WO 2012/064548.

In a preferred embodiment, the CHK1 inhibitor is LY2603618 or a pharmaceutically acceptable salt thereof. LY2603618 is also known 1-[5-bromo-4-methyl-2-[[(2S)-morpholin-2-yl]methoxy]phenyl]-3-(5-methylpyrazin-2-yl)urea or Rabusertib or and has the following chemical structure:

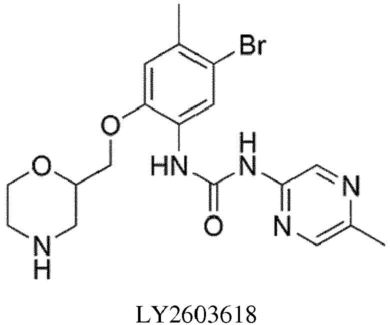

In a preferred embodiment, the CHK1 inhibitor is LY2606368 or a pharmaceutically acceptable salt thereof. LY2606368 is also known as 5-[[5-[2-(3-aminopropoxy)-6-methoxyphenyl]-1H-pyrazol-3-yl]amino]pyrazine-2-carbonitrile or Prexasertib and has the following chemical structure:

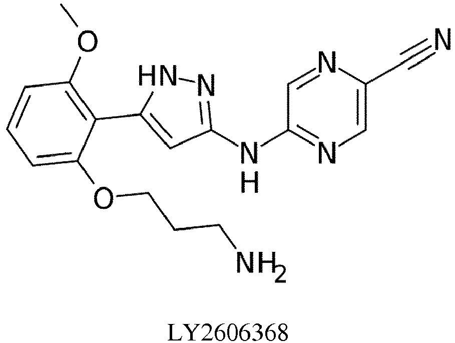

In yet another embodiment, the compound is a CHK1 inhibitor selected from a compound described in PCT International Publications WO 2007/044441, WO 2007/044449 and WO 2011/119457.

In a preferred embodiment, the CHK1 inhibitor is SCH900776/ MK-8776 or a pharmaceutically acceptable salt thereof. SCH900776/ MK-8776 is also known as (R)-(-)-6-Bromo-3-(1-methyl-1H-pyrazol-4-yl)-5-piperidin-3-yl-pyrazolo[1,5-a]pyrirnidin-7-ylamine and has the following chemical structure:

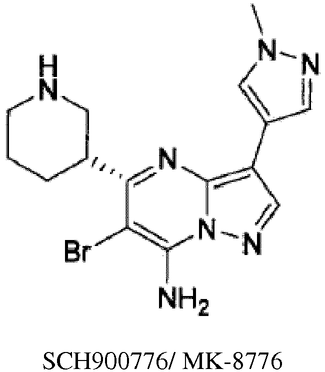

In still yet another embodiment, the compound is a CHK1 inhibitor selected from a compound described in PCT International Publication WO 2009/014637.

In a preferred embodiment, the CHK1 inhibitor is SCH900444 or a pharmaceutically acceptable salt thereof. SCH900444 is also known as 2-(1, 3-dihydro-5, 6-dimemoxy-2H-isoindol-2-yl)-N-[4-(1-piperazinyl)-3-pyridinyl]-4- pyrimidinecarboxamide and has the following chemical structure:

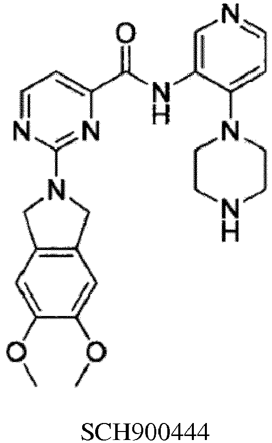

In still yet another embodiment, the compound is a CHK1 inhibitor selected from a compound described in PCT International Publication WO 2008/146035.

In a preferred embodiment, the CHK1 inhibitor is AZD-7762 or a pharmaceutically acceptable salt thereof. AZD-7762 is also known as 3-(carbamoylamino)-5-(3-fluorophenyl)-N-[(3S)-piperidin-3-yl]thiophene-2-carboxamide and has the following chemical structure:

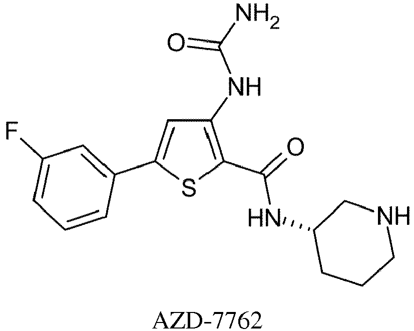

In still yet another embodiment, the compound is a CHK1 inhibitor selected from a compound described in PCT International Publications WO 2004/018419 and WO 2005/046589.

In a preferred embodiment, the CHK1 inhibitor is CHIR-124 or a pharmaceutically acceptable salt thereof. CHIR-124 is also known as 4-[[(3S)-1-azabicyclo[2.2.2]octan-3-yl]amino]-6-chloro-3-(1,3-dihydrobenzimidazol-2-ylidene)quinolin-2-oneand has the following chemical structure:

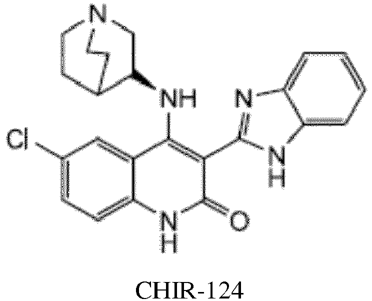

In still yet another embodiment, the compound is a CHK1 inhibitor selected from a compound described in PCT International Publications WO 2004/063198 and WO 2007/113671.

In a preferred embodiment, the CHK1 inhibitor is PF-477736 or a pharmaceutically acceptable salt thereof. PF-477736 is also known as (2R)-2-Amino-2-cyclohexyl-N-[2-(1-methyl-1H-pyrazol-4-yl)-6-oxo-5,6-dihydro-1H-[1,2]diazepino[4,5,6-cd]indol-8-yl]-acetamide and has the following chemical structure:

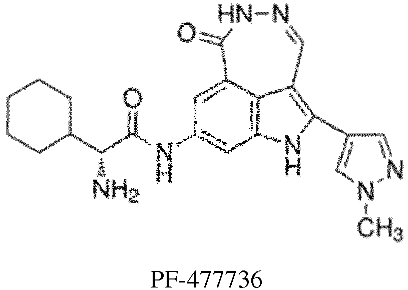

In a preferred embodiment, the CHK1 inhibitor is SRA737 or a pharmaceutically acceptable salt thereof. SRA737 is also known as (R)-5-((4-((morpholin-2-ylmethyl)amino)-5-(trifluoromethyl)pyridin-2-yl)amino)pyrazine-2-carbonitrile or CCT245737/PNT737 and has the following chemical structure:

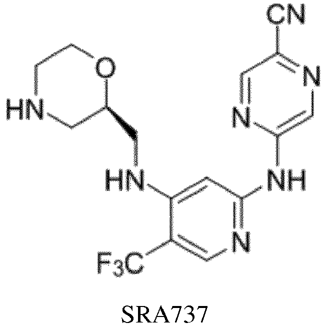

In still yet another embodiment, the compound is a CHK1 inhibitor selected from a compound described in PCT International Publications WO 2009/140320 and WO 2010/118390.

In a preferred embodiment, the CHK1 inhibitor is GDC-0575 or a pharmaceutically acceptable salt thereof. GDC-0575 is also known as RG7741.

In a particular embodiment, the CHK1 inhibitor is selected from the group consisting of LY2603618, LY2606368, SCH900776/MK-8776, SCH900444, AZD-7762, CHIR-124, PF-477736, SRA737 and GDC-0575.

### Cancers or tumors to be treated:

The terms "cancer", "cancerous", or "malignant" refer to or describe the physiological condition in mammals that is typically characterized by unregulated cell growth. Examples of cancer include, for example, leukemia, lymphoma, blastoma, carcinoma and sarcoma.

Various cancers are also encompassed by the scope of the invention, including, but not limited to, the following: carcinoma including that of the bladder (including accelerated and metastatic bladder cancer), breast, colon (including colorectal cancer), kidney, liver, lung (including small and non-small cell lung cancer and lung adenocarcinoma), ovary, prostate, testis, genitourinary tract, lymphatic system, rectum, larynx, pancreas (including exocrine pancreatic carcinoma), esophagus, stomach, gall bladder, cervix, thyroid, and skin (including squamous cell carcinoma); hematopoietic tumors of lymphoid lineage including leukemia, acute lymphocytic leukemia, acute lymphoblastic leukemia, B-cell lymphoma, T-cell lymphoma (including cutaneous or peripheral T-cell lymphoma), Hodgkins lymphoma, non-Hodgkins lymphoma, hairy cell lymphoma, histiocytic lymphoma, and Burketts lymphoma; hematopoietic tumors of myeloid lineage including acute and chronic myelogenous leukemias, myelodysplastic syndrome, myeloid leukemia, and promyelocytic leukemia; tumors of the central and peripheral nervous system including astrocytoma, neuroblastoma, glioma, and schwannomas; tumors of mesenchymal origin including fibrosarcoma, rhabdomyosarcoma, and osteosarcoma; other tumors including melanoma, xenoderma pigmentosum, keratoactanthoma, seminoma, thyroid follicular cancer, and teratocarcinoma; melanoma, unresectable stage III or IV malignant melanoma, squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatocarcinoma, breast cancer, colon carcinoma, and head and neck cancer, retinoblastoma, gastric cancer, germ cell tumor, bone cancer, bone tumors, adult malignant fibrous histiocytoma of bone; childhood malignant fibrous histiocytoma of bone, sarcoma, pediatric sarcoma; myelodysplastic syndromes; neuroblastoma; testicular germ cell tumor, intraocular melanoma, myelodysplastic syndromes; myelodysplastic/myeloproliferative diseases, synovial sarcoma.

In a preferred embodiment of the present invention, the cancer is a solid tumor. For instance, the cancer may be sarcoma and osteosarcoma such as Kaposi sarcome, AIDS-related Kaposi sarcoma, melanoma, in particular uveal melanoma, and cancers of the head and neck, kidney, ovary, pancreas, prostate, thyroid, lung, esophagus, breast in particular triple negative breast cancer (TNBC), bladder, colorectum, liver and biliary tract, uterine, appendix, and cervix, testicular cancer, gastrointestinal cancers and endometrial and peritoneal cancers. Preferably, the cancer may be sarcoma, melanoma, in particular uveal melanoma, and cancers of the head and neck, kidney, ovary, pancreas, prostate, thyroid, lung, esophagus, breast in particular (TNBC), bladder, colorectum, liver, cervix, and endometrial and peritoneal cancers.

The pharmaceutical compositions and the products, kits or combined preparations described in the invention may be useful for inhibiting the growth of solid tumors, decreasing the tumor volume, preventing the metastatic spread of tumors and the growth or development of micrometastases, preventing the tumor recurrence and preventing the tumor relapse. The pharmaceutical compositions and the products, kits or combined preparations described in the invention are in particular suitable for the treatment of poor prognosis patients or of radio- or chemo-resistant tumors. In a particular embodiment, the cancer is a high-grade or advanced cancer or is a metastatic cancer.

### Regimen, dosages and administration routes:

The effective dosage of each of the combination partners employed in the combined preparation of the invention may vary depending on the particular compound or pharmaceutical composition employed, the mode of administration, the condition being treated, the severity of the condition being treated. Thus, the dosage regimen of the combined preparation of the invention is selected in accordance with a variety of factors including the route of administration and the patient status. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

The invention more particularly relates to a pharmaceutical composition, a kit, product or combined preparation wherein the amount or dosage of belinostat can be lowered in comparison with its amount or dosage when it is used alone. Indeed, the combination of belinostat and an ATRi or the combination of belinostat and a CHK1i lead to a clear synergistic effect of the two active ingredients. This potentiating effect allows the decrease of the amount of the belinostat, which generally exhibit a toxicity for the normal cells and therefore can be associated with adverse effects. Then, with the combined treatment of the invention, it is possible to preserve the efficacy of the treatment, or even to improve it, while decreasing its adverse effects, in particular the adverse effects of belinostat.

Alternatively, instead of lowering the amount or dosage of the belinostat, the administration frequency of belinostat or its or treatment period can be reduced.

The invention also relates to a method for treating cancer, to a pharmaceutical composition, to a product, kit or combined preparation as disclosed above, wherein the amounts of belinostat and a DDR cell cycle checkpoint inhibitor selected from the group consisting of ATRi and CHK1i in the combined preparation are such that the combined therapeutic effect of the two active ingredients is synergistic.

By the term "synergistic" therapeutic effect is meant that the obtained therapeutic effect of the combination is more than the addition of the therapeutic effect of each partner alone (i.e. more than the effect of belinostat alone plus the effect of the DDR cell cycle checkpoint inhibitor selected from the group consisting of ATRi and CHK1i alone).

The invention relates to a method for the treatment of a cancer, to a pharmaceutical composition, to a product, kit or combined preparation as disclosed above, wherein belinostat can be used at the same dosage or at lower dosage than the conventional dosage used in chemotherapy for the same indication and the same administration route when it is used alone (i.e., an amount equal to or preferably lower than the one used in conventional chemotherapy), also called herein a sub-therapeutic amount.

More particularly, the amount can be for instance 100, 90, 80, 70, 60, 50, 40, 30, 20 or 10 % of the conventional therapeutic dosage (in particular for the same indication and the same administration route). The conventional therapeutic dosages are those acknowledged by the drug approvals agencies (e.g., FDA or EMEA). In that respect, the present invention relates to a method for the treatment of a cancer, to a pharmaceutical composition, to a product, kit or combined preparation as disclosed above, wherein the amount of the belinostat is used at a sub-therapeutic dosage and the amount of a DDR cell cycle checkpoint inhibitor selected from the group consisting of ATRi and CHK1i is such that the combined therapeutic effect of the two active ingredients is synergistic.

Determining a synergistic interaction between two components, the optimum range for the effect and absolute dose ranges of each component for the effect may be definitively measured by administration of the components over different w/w ratio ranges and doses to patients in need of treatment. For humans, the complexity and cost of carrying out clinical studies on patients may render impractical the use of this form of testing as a primary model for synergy. However, the observation of synergy in one species can be predictive of the effect in other species and animal models exist to measure a synergistic effect and the results of such studies can also be used to predict effective dose and plasma concentration ratio ranges and the absolute doses and plasma concentrations required in other species by the application of pharmacokinetic/pharmacodynamic methods. Correlations between cancer models and effects seen in man suggest that observed synergy on animal models may be predictive of a synergy on man too.

The pharmacological activity of a combination of the invention may, for example, be demonstrated in a clinical study or more preferably in a test procedure. Suitable clinical studies are, for example, open label non-randomized, dose escalation studies in patients with advanced tumors. Such studies can prove the synergism of the active ingredients of the combination of the invention. The beneficial effects on proliferative diseases can be determined directly through the results of these studies or by changes in the study design which are known as such to a person skilled in the art. Such studies are, in particular, suitable to compare the effects of a monotherapy using the active ingredients and a combination of the invention. Preferably, the combination partner (a) is administered with a fixed dose and the dose of the combination partner (b) is escalated until the maximum tolerated dosage is reached. Alternatively, the combination partner (b) is administered with a fixed dose and the dose of the combination partner (a) is escalated until the maximum tolerated dosage is reached.

The treatment may include one or several cycles, for instance two to ten cycles, in particular two, three, four or five cycles. The cycles may be continued or separated. For instance, each cycle is separated by a period of time of one to eight weeks, preferably three to four weeks.

### Biomarkers:

In some embodiments, one or more biomarkers may be used to monitor or determine the efficacy of the treatment. In certain embodiments, the percentage of cancer cells with micronuclei (MN) in paired samples may be used as a biomarker; micronuclei is believed to correlate with the level of the inhibition of HDAC activity. For instance, in some embodiments, the percentage of cancer cells with micronuclei in a tumor biopsy may be used to determine the efficacy of monotherapy with belinostat or combination therapy including a ATRi or a CHK1i in a subject. In some such embodiments, a first tumor biopsy may be taken between about one to about three hours (e.g., two hours) before administration of belinostat and a second tumor biopsy may be taken about several hours (e.g., twelve hours) after administration of belinostat. The percentage of cancer cells with micronuclei in the first biopsy is set to be 100%, such that the percentage of cancer cells with micronuclei in the second biopsy is normalized by the amount in the first biopsy.

Criteria for identifying micronuclei are well-established, for instance as described in Countryman & Heddle (1976, Mutation Research, 41, 321-332). In particular, the criteria are the followings:
- diameter less than 1/3rd the main nucleus;
- non-refractility (to exclude small stain particles);
- colour same as or lighter than the nucleus (to exclude large stain particles);
- location within 3 or 4 nuclear diameters of of a nucleus; and not touching the nucleus (to make frequency measurements meaningful); and
- no more than 2 micronuclei associated with one nucleus.

The counting of the number of cancer cells having micronuclei can be determined under microscope after appropriate staining. The micronuclei are clearly smaller than the cell nuclei and are physically separate from them. The counting can be carried out by visual scoring or by automated scoring procedures. Prototypes of software for automated image analysis have been described in the literature since the early 1990s and developed by Becton-Dickinson and Loats Associates, Inc (LAI Automated Micronucleus Assay System;. 201 East Main St. Westminster, MD 21157 410-876-8055).

In one embodiment, the method for determining the frequency of cancer cells with micronuclei comprises lysing cells of the sample to release nuclei and micronuclei, counting the nuclei and micronuclei, and determining the frequency of cancer cells with micronuclei. The lysis can be achieved by any known procedures, such as those described in Nusse and Marx (1997, Mutat Res. 392(1-2): 109-15), the disclosure of which being incorporated by reference. The nuclei and micronuclei can be analyzed by flow cytometry. When nuclei and micronuclei are analyzed by flow cytometry, the nuclei and micronuclei are stained with a dye prior to flow cytometry.

Analysis of nuclei and micronuclei can be performed by flow cytometry as described in Nusse and Marx (1997, Mutat Res. 392(1-2):109-15), the disclosure of which being incorporated by reference. In this embodiment, nuclei may be distinguished from micronuclei by their greater light scatter and greater DNA content. The frequency of cells with micronuclei is calculated as the micronuclei frequency per nuclei.

The micronuclei and nuclei can be stained by any appropriate dye. In a preferred embodiment, the dye is a fluorescent dye. For instance, the florescent dye can be selected from the group consisting of DAPI (4',6-diamidino-2-phenylindole), propidium iodide, Hoechst 33342, carboxyfluorescein diacetate succinimidyl ester (CFSE) and dyes of the PKH series. When nuclei and micronuclei are analyzed by flow cytometry, the dye can be selected from the group consisting of DAPI (4',6-diamidino-2-phenylindole), propidium iodide and Hoechst 33342. It will be appreciated by those skilled in the art that a number of alternative nucleic acid dyes may also be used.

For instance, a method for determining the frequency of micronuclei can comprise:
- incubating cells from the sample for a sufficient time to allow a substantial population of cells to complete at least one cellular division;
- optionally, sorting the cells to obtain a substantially pure population of cells which have undergone one cellular division;
- lysing the cells to release nuclei and micronuclei; and
- determining the nuclei and micronuclei in order to determine the frequency of micronuclei.

Based on the frequency of cancer cells with micronuclei, it is possible to monitor or determine the efficacy of the treatment of the tumor to a treatment with belinostat or with combination therapy of the invention. An increase in the frequency of cancer cells with micronuclei between the first tumor biopsy and the second tumor biopsy is indicative that the treatment with belinostat monotherapy or with combination therapy of the invention is effective.

In other embodiments, the level of gamma-H2AX in paired samples may be used as a biomarker; gamma H2AX is believed to correlate with the level of the inhibition of HDAC activity. For instance, in some embodiments, the level of gamma-H2AX in a tumor biopsy may be used to determine the efficacy of monotherapy with belinostat or combination therapy including a ATRi or a CHKi in a subject. In some such embodiments, a first tumor biopsy may be taken between about one to about three hours (e.g., two hours) before administration of belinostat and a second tumor biopsy may be taken about several hours (e.g., twelve hours) after administration of belinostat. The level of gamma-H2AX in cancer cells in the first biopsy is set to be 100%, such that level of gamma-H2AX in the second biopsy is normalized by the amount in the first biopsy.

In one embodiment, the method for determining the level of gamma-H2AX may be performed by using an antibody raised against gamma-H2AX which can therefore be visualized by immunofluorescence through secondary antibodies. The detection and visualization of gamma-H2AX by flow cytometry allow to monitor or determine the efficacy of the treatment of the tumor with belinostat or with combination therapy of the invention. An increase in the level of gamma-H2AX between the first tumor biopsy and the second tumor biopsy is indicative that the treatment with belinostat monotherapy or with combination therapy of the invention is effective.

Further aspects and advantages of the present invention will be described in the following examples, which should be regarded as illustrative and not limiting.

### Examples

### EXAMPLE 1: Efficacy of the combined treatment belinostat and ATR inhibitors - In vitro test on cell survival

### Materials and Methods

### Cell culture

Cell cultures were performed with the triple negative breast cancer (TBNC) cell line MDA-MB-231, the histiocytic lymphoma cell line U937 and the non-tumor mammary cell line MCF-10A. Cells were grown according to the supplier's instructions. Cell lines were maintained at 37°C in a humidified atmosphere at 5% CO₂, except the MDA-MB-231 cell line which was maintained at 0% CO₂.

### Drugs treatment, measurement of cellular survival and synergy analysis

In a first step, the characterization of the effect of drugs monotherapy on cell survival (tumour and non-tumour cells) was performed. MDA-MB-231 (5.10³ cells/well), MCF-10A (5.10³ cells/well) and U937 (2.10⁴ cells/well) were seeded in 96 well-plates and incubated 24 hours at +37°C before addition of the drug at increasing concentrations for 4 to 7 days. Following drug exposure, cell survival was measured using the XTT assay (Sigma Aldrich). Briefly, the XTT solution was added directly to each well containing cell culture and the cells incubated for 4 to 24 hours at 37°C before reading the absorbance at 490 nm and 690 nm using a microplate reader (BMG Fluostar, Galaxy). Cell survival is calculated as the ratio of living treated cells to living mock-treated cells. The IC50 (which represents the dose at which 50% of the cells are viable) is calculated by a non-linear regression model using GraphPad Prism software (version 5.04) by plotting the percentage viability against the Log of the drug concentration on each cell line.

In a second step, analysis and quantification of the drug combination efficacy were performed. Cells were seeded in 96-well plates at the appropriate densities and treated with belinostat (PXD-101 - Spectrum) and/or ATR (ATRi - AZD6738, Selleckchem; VE-821, Selleckchem) simultaneously during 4 to 7days. Belinostat + ATRi combinations was performed at a constant ratio (from IC50/8 to IC50*8) as previously described (Chou TC, Talalay P. Adv Enzyme Regul. 1984;22:27-55 (Quantitative analysis of dose-effect relationships: the combined effects of multiple drugs or enzyme inhibitors). Cell survival was measured using the XTT assay (see above).

Combination efficacy is evaluated by "the combination index (CI)" which is determined using the CalcuSyn software tool based on the Chou Talalay method. A CI could be interpreted as follows: CI<1, =1, and >1 indicate synergism; additive effect and antagonism, respectively (CalculSyn manual, Biosoft, 2006). Note that we define synergism as a more than expected additive effect, and antagonism as a less than expected additive effect.

### Statistical analysis

All statistical analysis are performed with a two-tailed Student t test.

### Results

The inventors tested the effect of the combination of belinostat with ATR inhibitors on the survival of cancer cells as well as on normal cells (non-cancer cells).
More particularly, the results are shown in **Figure 1****.**

Tumor cells (MDA-MB-231, U937) were treated with belinostat (light grey), ATRi (dark grey) or both (black) at doses corresponding to the IC50 (**Table 1**)**.** The same drug doses as MDA-MB-231 were applied to MCF-10A non-tumor cells. Survival was measured 4 days (U937) or 7 days (MDA-MB-231 and MCF-10A) after treatment using the XTT assay. Results are presented as % to non-treated cells. In tumor cells, where monotherapy with belinostat or ATRi resulted in a cell survival of 40 to 70% (compared to non-treated cells), combined treatments belinostat+ATRi decreased significantly cell survival (**Figure 1****, left panel**)**.** The non-tumor cells MCF-10A were insensitive to belinostat. However, they showed a sensitivity to the ATRi AZD6738. Adding belinostat didn't increase the sensitivity of non-tumor cells to AZD6738. Synergy analysis revealed a synergistic efficacy of the combined treatment belinostat+ATRi on MDA-MB-231 and U937 cell lines (CI<1), compared to non-tumor cells (**Figure 1****, right panel**)**.** Interestingly, a high synergy between belinostat and ATRi was observed even at very low doses, making the combination very attractive for a clinical investigation (data not shown). Absence of any effect in non-tumor cells predicts a non-toxicity and a high safety of this combined treatments in normal tissues.

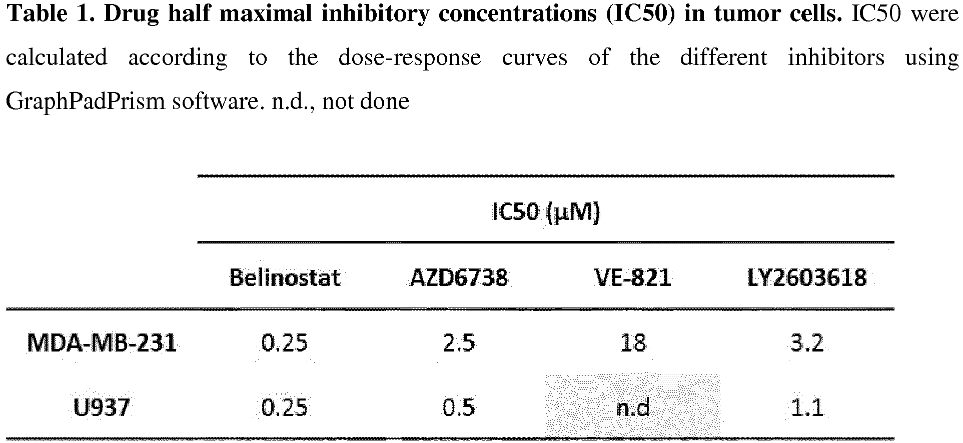

### EXAMPLE 2: Efficacy of the combined treatment belinostat and CHK1 inhibitors - In vitro test on cell survival

### Materials and Methods

### Cell culture

Cell cultures were performed with the triple negative breast cancer cell (TBNC) line MDA-MB-231, the histiocytic lymphoma cell line U937 and the non-tumor mammary cell line MCF-10A. Cells were grown according to the supplier's instructions. Cell lines were maintained at 37°C in a humidified atmosphere at 5% CO₂, except the MDA-MB-231 cell line which was maintained at 0% CO₂.

### Drugs treatment, measurement of cellular survival and synergy analysis

In a first step, the characterization of the effect of drugs monotherapy on cell survival (tumour and non-tumour cells) was performed. MDA-MB-231 (5.10³ cells/well), MCF-10A (5.10³ cells/well) and U937 (2.10⁴ cells/well) were seeded in 96 well-plates and incubated 24 hours at +37°C before addition of the drug at increasing concentrations for 4 to 7 days. Following drug exposure, cell survival was measured using the XTT assay (Sigma Aldrich). Briefly, the XTT solution was added directly to each well containing cell culture and the cells incubated for 4 to 24 hours at 37°C before reading the absorbance at 490 nm and 690 nm using a microplate reader (BMG Fluostar, Galaxy). Cell survival is calculated as the ratio of living treated cells to living mock-treated cells. The IC50 (which represents the dose at which 50% of the cells are viable) is calculated by a non-linear regression model using GraphPad Prism software (version 5.04) by plotting the percentage viability against the Log of the drug concentration on each cell line.

In a second step, analysis and quantification of the drug combination efficacy were performed. Cells were seeded in 96-well plates at the appropriate densities and treated with belinostat (PXD-101 - Spectrum) and CHK1 inhibitors (CHK1i - LY2603618, Selleckchem) simultaneously during 4 to 7 days. Belinostat + CHK1i combinations is performed at a constant ratio (from IC50/8 to IC50*8) as previously described¹. Cell survival is measured using the XTT assay (see above).

Combination efficacy is evaluated by "the combination index (CI)" which was determined using the CalcuSyn software tool based on the Chou Talalay method. A CI could be interpreted as follows: CI<1, =1, and >1 indicate synergism; additive effect and antagonism, respectively (CalculSyn manual, Biosoft, 2006). Note that we define synergism as a more than expected additive effect, and antagonism as a less than expected additive effect.

### Statistical analysis

All statistical analysis were performed with a two-tailed Student t test.

### Results

The inventors tested the effect of the combination of belinostat with CHK1 inhibitors on the survival of cancer cells as well as on normal cells (non-cancer cells).
More particularly, the results are shown in **Figure 2****.**

Tumor cells (MDA-MB-231, U937) were treated with belinostat (light grey), CHK1i (dark grey) or both (black) at doses corresponding to the IC50 (**Table 1**)**.** The same drug doses as MDA-MB-231 were applied to MCF-10A non-tumor cells. Survival was measured 4 days (U937) or 7 days (MDA-MB-231 and MCF-10A) after treatment using the XTT assay. Results are presented as % to non-treated cells. In tumor cells, where monotherapy with belinostat or CHK1i resulted in a cell survival of 40 to 60% (compared to non-treated cells), combined treatments Belinostat+CHK1i decreased significantly cell survival (**Figure 2****, left panel**)**.** The non-tumor cells MCF-10A were insensitive to belinostat. However, they showed a sensitivity to the CHK1i LY2603618. Adding belinostat didn't increase the sensitivity of non-tumor cells to CHK1i. Synergy analysis revealed a synergistic efficacy of the combined treatment belinostat+ CHK1i on MDA-MB-231 and U937 cell lines (CI<1), compared to non-tumor cells (**Figure 2****, right panel**)**.** Interestingly, a high synergy between belinostat and CHK1i was observed even at very low doses, making the combination very attractive for a clinical investigation (data not shown). Absence of any effect in non-tumor cells predicts a non-toxicity and a high safety of this combined treatments in normal tissues.

## Claims

1. A pharmaceutical composition or a kit (kit-of-parts) comprising belinostat and a DNA damage response (DDR) cell cycle checkpoint inhibitor selected from the group consisting of an ATR inhibitor (ATRi) and a CHK1 inhibitor (CHK1i).

2. The pharmaceutical composition or the kit (kit-of-parts) according to claim 1, wherein the DDR cell cycle checkpoint inhibitor is an ATRi.

3. The pharmaceutical composition or the kit (kit-of-parts) according to claim 2, wherein the ATRi is selected form the group consisting of VE-821, VE-822/VX-970, AZD-6738, AZ20, and BAY-1895344.

4. The pharmaceutical composition or the kit (kit-of-parts) according to claim 1, wherein the DDR cell cycle checkpoint inhibitor is a CHK1i.

5. The pharmaceutical composition or the kit (kit-of-parts) according to claim 4, wherein the CHK1i is selected from the group consisting of LY2603618, LY2606368, SCH900776/MK-8776, SCH900444, AZD-7762, CHIR-124, PF-477736, SRA737 and GDC-0575.

6. A pharmaceutical composition according to any one claims 1 to 5 for use in the treatment of a cancer.

7. A kit (kit-of-parts) comprising belinostat and a DDR cell cycle checkpoint inhibitor selected from the group consisting of an ATRi and a CHK1i and as a combined preparation for simultaneous, separate or sequential use, in particular for use in the treatment of cancer.

8. The pharmaceutical composition or the kit for use according to claim 7, wherein the cancer is selected from leukemia, lymphoma, sarcoma, melanoma, and cancers of the head and neck, kidney, ovary, pancreas, prostate, thyroid, lung, esophagus, breast, bladder, brain, colorectum, liver, and cervix.

9. The pharmaceutical composition or the kit for use according to claim 8, wherein the cancer is triple-negative breast cancer (TNBC).
